# EUROPEAN PATENT SPECIFICATION

(11) **EP 4 536 797 B1**
(45) Date of publication and mention of the grant of the patent: **08.07.2026**
(21) Application number: 23739802.9
(22) Date of filing: 09.06.2023
(51) Int. Cl.: C12M 1/107, C12M 1/00, C12M 1/12

(54) **ANAEROBIC DIGESTER**
ANAEROBER FAULBEHÄLTER
DIGESTEUR ANAÉROBIE

(30) Priority: 10.06.2022 ZA 202206552
(43) Date of publication of application: 16.04.2025
(73) Proprietor: University of South Africa, 0002 Pretoria (ZA)
(72) Inventor: MATAMBO, Sylvester Tonderayi, 2188 Johannesburg (ZA); IJOMA, Nkechinyere Grace, 1459 Johannesburg (ZA); RASHAMA, Charles, 0002 Pretoria (ZA); MUTUNGWAZI, Asheal, 1709 Johannesburg (ZA)
(74) Representative: Hanna, John Philip
(86) International application number: PCT/ZA2023/050030
(87) International publication number: WO 2023/240297

(56) References cited:
- CN-U- 201 801 518
- GB-A- 1 522 780
- US-A- 4 401 441

## Description

### FIELD OF INVENTION

This invention relates to an anaerobic digester assembly as defined in independent claim 1, and in particular disclosing a single compartment anaerobic digestion reaction chamber.

### BACKGROUND TO THE INVENTION

Household, agricultural and food processes usually generate solid and/or liquid waste streams that have dissolved or suspended organic solids. Such waste poses a significant environmental disposal problem.

As is well understood, anaerobic digestion has essentially four stages, namely:
Hydrolysis - in which particulates are solubilised and substances such as carbohydrates being converted into sugars, fats into fatty acids and proteins into amino acids;
Acidogenesis - in which simple monomers are converted into volatile fatty acids;
Acetogenesis - in which volatile fatty acids are converted into acetic acid, carbon dioxide and hydrogen; and
Methanogenesis - in which the products of the preceding processes, such as acetic acid are converted into methane, carbon dioxide and water.

Anaerobic digesters include both batch and continuous digesters. A continuous process is usually favoured, since the waste is processed continuously, and there is a steady supply of methane. The classic design for industrial digesters is a variant of a one stage digester, the continuously stirred tank reactor ("CSTR"). In a CSTR digester, all contents are completely mixed. Thus the effluent will contain some amount of freshly added, undigested waste material, and will include some active microbes.

The CSTR is usually used for waste with a medium solids content, from 2 to 10% dry matter. Two alternative designs to overcome these problems are the "plug-flow" digester and the microbe retention digester. In a plug-flow digester, the waste passes through the digester in a sequential manner from the inlet to the outlet.

The name "plug-flow" is usually used for designs that are unstirred and tubular. The solid material tends to move through the digester sequentially, while the liquid fraction mixes more rapidly. The retention digester is designed to retain the microorganisms in the digester.

Some anaerobic digesters are considered two-stage digesters, because the processes of hydrolysis and acidification are separated from the processes of acetification and methanogenesis. This separation usually produces methane gas with lower levels of impurities.

Digesters today have no exact classification, however certain names are used in the industry including FRP (Fibre glass reinforced unsaturated polyester resin), plastic soft (PS) digesters, and plastic hard (PH) digesters. PS digesters are also called bag digesters in some countries because they look like big soft bags. Materials applied include soft polyvinyl chloride (PVC), red mud, polymethyl methacrylate, low-density PE, PE, and polypropylene (PP). PH digesters are normally hard digesters. In contrast to soft digesters, PH digesters are made out of hard PVC, ABS, PE, PP, high-density polyethylene (HDPE), and linear low-density PE.

In most digesters, the temperature is controlled. The methane producing microorganisms (methanogens) dictate the optimum temperature for the digester to operate efficiently. Two common temperature ranges of digesters are a mesophilic temperature range (20 °C to 45 °C) or a thermophilic temperature range (50 °C to 65 °C). Methane production decreases if the digester operating temperature is sub-optimal or exceeds the optimal temperature ranges required by the methanogenic microorganisms.

Most conventional digesters today, if not all, are compartmentalised i.e., they have a digestion section which is divided into compartments in an attempt to separate the biochemical processes (hydrolysis, acidogenesis, acetogenesis and methanogenesis) in one way or another. This sometimes improves efficiency, but dramatically adds to the digester production costs and complexity of machine needed to manufacture same.

Due to its construction, a polyester composite material never loses its waterproof characteristic, cutting down on replacement or expensive treatments. It provides stronger protection against oils and chemicals than does for example acrylic fabric.

Polyester's overall durability, especially protection against abrasion, is also unparalleled. The monomer vinyl chloride is an organochloride which in combination forms the polymer, polyvinyl chloride (PVC). Although, flexible and capable of expansion, it is unable to withstand on a long-term basis UV deterioration and heat and may experience cracking due to weathering.

To compensate for the reduced ability of the conventional polyvinyl chloride (PVC) bags in withstanding the long term effect of ultraviolet radiations which is an eventual deterioration observed in previous designs after long-term exposure. This invention integrates a two-layer approach, where the inner bag is PVC and the outer reinforcement bag is composed of tarp-type polyester coated vinyl material.

Complete pre-fabricated digesters, or the materials used for construction are imported, from mainly China. Importations severely increase digester construction costs and delays.

CN201801518U discloses a device for anaerobic fermentation, comprising a rectangular trough body of concrete material with a sealed top cover comprising a flexible membrane. A mechanical stirring device is installed inside the trough body, a feed port is provided at one end connected to a delivery pump through a piping, and a discharge device is provided at the other end for discharge by suction.

As such, the invention focuses on providing anaerobic digesters, factoring in these current impediments and improving others to make the production of digesters more economical for the African and more specifically for the South African setting.

Moreover, with most conventional digesters, if fouling occurs, cleaning of the digesters is quite difficult to execute.

### OBJECT OF THE INVENTION

It is an object of the invention to provide for a single compartment anaerobic digestion (AD) reaction chamber for the treatment of aqueous organic waste in which at least some of the aforementioned disadvantages are reduced or obviated.

### SUMMARY OF THE INVENTION

In accordance with the invention there is provided a anaerobic digester (AD) assembly as defined in claim 1.

The resiliently flexible top member may be a planar, substantially flat pliable/flexible member.

The anaerobic digestion assembly may comprise sealing means for providing and enhancing airtightness between the resiliently flexible top member and the base member.

The sealing means may comprise a first seal member and a second seal member, and the resiliently flexible top member may operatively be sandwiched between the first seal member and second seal member.

The anaerobic digestion assembly may comprise securing means for securing the resiliently flexible top member, once it has been sandwiched between the first and second seal members, and the base member to each other.

A drainage hole may be defined by the base member.

The drainage hole may be disposed near the waste outlet.

The base member may be inclined relative to the horizontal, preferably at an angle of 2 degrees to the horizontal. In particular, a base of the base member may be inclined relative to the horizontal from an inlet side to an outlet side at an angle of at least 2 degrees to the horizontal.

The base member may have a seat the for accommodating the sealing means .

The seat may project laterally outwardly from the periphery or near the periphery or the sides of the base member proximate the upper edge of the base member.

The seat may be in the form of a flange, in particular an outwardly curved flange.

The assembly may have an organic waste holding/receiving receptacle in communication with the waste inlet for feeding organic waste into the base member.

The organic waste holding receptacle may be flush or integrally connected with an end wall of the base member defining the inlet.

The organic waste holding receptacle may have a lid.

The assembly may comprise a digestate holding receptacle that is in communication with the outlet.

The digestate holding receptacle may be flush or integrally connected with the end wall defining the outlet.

The digestate holding receptacle may have a lid.

The assembly may further comprise attachment means including at least one bracket for allowing a greenhouse covering support structure to be attached and secured thereto, wherein the greenhouse covering support is for trapping heat generated from the digester and enabling an increase in temperatures in the digester for efficient digestion.

The at least one bracket may be provided at each corner of the base member.

The at least one bracket may be secured/fastened at each corner of the base member for enabling attachment of a removable greenhouse cover.

The base member is made from a material comprising a thermoplastic polymer, including but not limited to Polypropylene (PP), Poly Vinyl Chloride (PVC) or High Density Polyethylene (HDPE) or the like, preferably HDPE.

The resiliently flexible top member may be made from a composite material comprising a polyester and a vinyl, and the composite material may have been treated for acid and UV resistance.

The composite material may be a good heat conductor arranged to absorb and transmit solar energy into the base member.

The first seal member may be a first gasket and the second seal member may be a second gasket.

The first seal member may be arranged on the base member, in particular accommodated in the seat of the base member, and the second sealing may be disposed on the resiliently flexible top member to ensure airtightness between the top member and the base member .

The first and second gaskets may be in the form of rubber gaskets.

The assembly may further comprise a frame that is arranged to abut the second seal member that is disposed on the top member. The frame may be a metallic frame, in particular a stainless steel frame.

The securing means may be arranged to secure the frame, first seal member, the top member and the second seal member to the bottom member.

The securing means may comprise a threaded bolt arranged to pass through aligned openings of the sequential arrangement of the metallic frame, second seal member, top member, first seal member, and the base member, and the securing means may further comprise a first nut for securing the bolt to the frame and a second nut for securing the bolt to the base member.

### BRIEF DESCRIPTION OF THE DRAWINGS

The invention will now be described in more detail, by way of example only, with reference to the accompanying drawings in which:
**FIGURE 1****:** shows an exploded view of the anaerobic digester assembly in accordance with the invention;
**FIGURE 2****:** shows a perspective view of a base member of a reactor vessel of an anaerobic digester assembly in accordance with the invention;
**FIGURE 3****:** shows a perspective view of an assembled anaerobic digester in accordance with the invention;
**FIGURE 4****:** shows a perspective view of an assembled anaerobic digester in accordance with the invention when in use;
**FIGURE 5****:** shows a cross-sectional detailed view of an arrangement of a sealing means, top and bottom members of the anaerobic digester assembly in accordance with the invention;

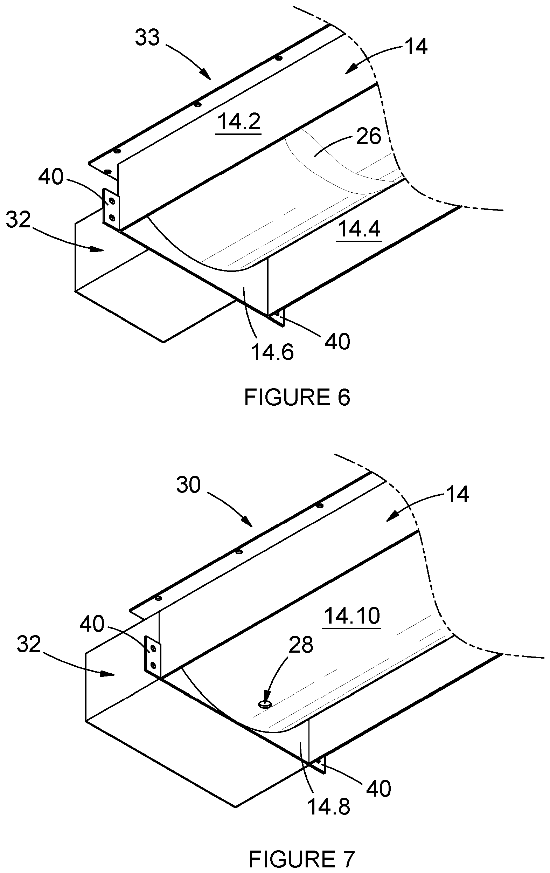

**FIGURE 6****:** shows a bottom view of an inlet side of the base member; and
**FIGURE 7****:** shows a bottom view of an outlet side of the base member.

### DETAILED DESCRIPTION OF THE INVENTION

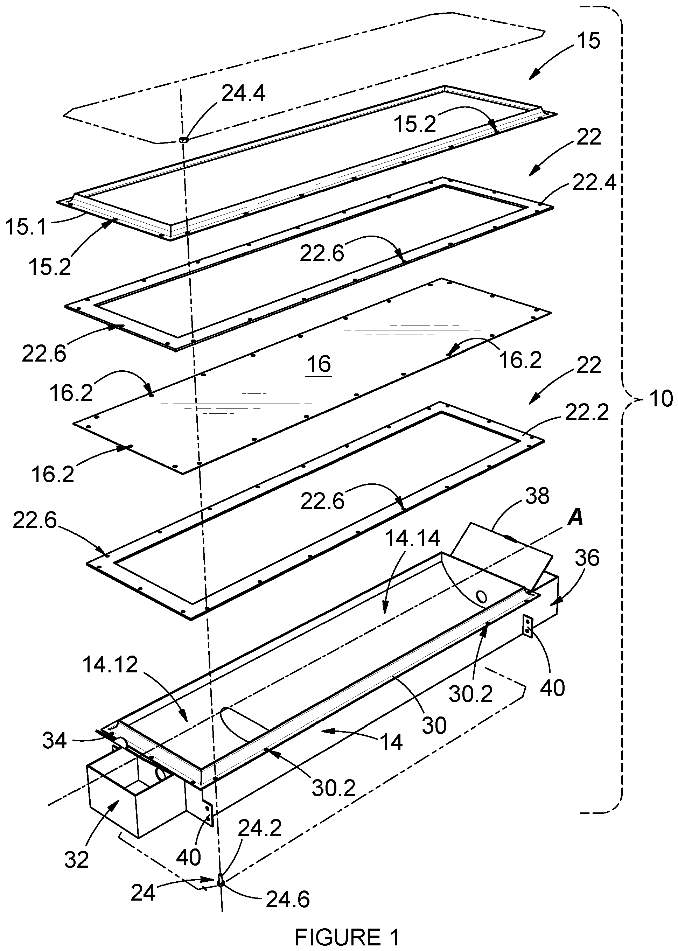

With reference to Figure 1, there is provided an anaerobic digester assembly 10 for anaerobically digesting aqueous organic waste (not shown). The anaerobic digester comprises a horizontally arranged elongate reactor vessel 12 that has a longitudinal axis A-A. The anaerobic digester assembly 10 comprises a base member 14 and top member 16.

The base member 14 comprises a pair of opposite first and second side walls 14.2 and 14.4 respectively, a pair of opposite first and second end walls 14.6 and 14.8 respectively, and a hemispherical, downwardly curved base 14.10. The base 14.10 may be inclined relative to the horizontal from the first end wall 14.6 side to the second end wall 14.8 side. The angle of inclination relative to the horizontal may at least be about 2 degrees.

The base member 14 defines an upper opening 14.12 that is arranged to be covered by the top member 16, in use. The base member 14 further defines a single hollow/compartment 14.14 that is arranged to accommodate aqueous organic waste.

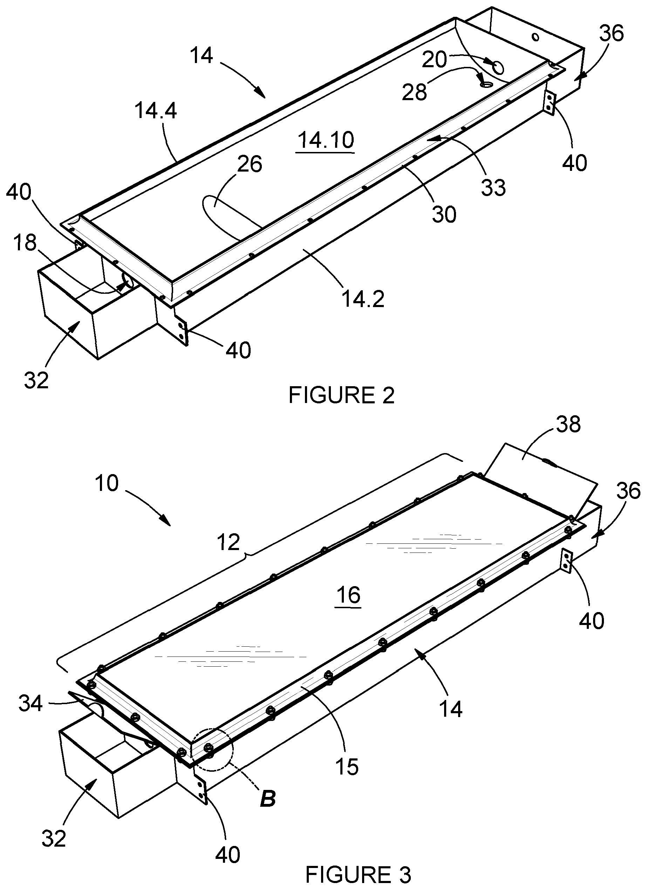

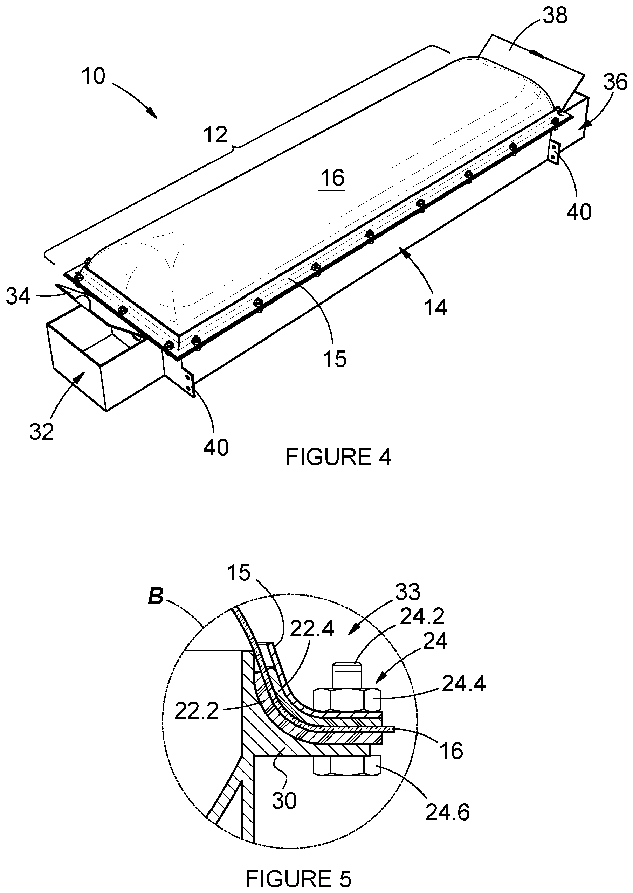

The first end wall 14.6 of the base member 14 defines an organic waste inlet 18 and a digestate outlet 20, as shown in Figure 2. The base member 14 further comprises a seat 30 that projects laterally outwardly proximate an upper edge of each of the side walls 14.2, 14.4 and end walls 14.6, 14.8. As shown in most of the Figures, the seat 30 is rectangular and continuously extends around the upper periphery of the base member 14. In the present embodiment, as shown in Figure 5, the seat 30 is in the form of a downwardly curved flange defining a channel 33 and a series of flange openings 30.2 on an outer lip thereof.

The base member 14 is made from a thermoplastic polymer selected from polypropylene, polyvinyl chloride or High Density Polyethylene (HDPE) or the like. In the present embodiment, the base member is made from HDPE. The top member 16 is in the form of a planar member made from a pliable/flexible material. As shown in Figure 1, the top member 16 is rectangular in shape. As mentioned above, the top member 16 is arranged to cover the upper opening 14.12 of the base member 14 so as to isolate the hollow 14.14 from the external environment and thereby avoid contaminants or foreign matter from being introduced in the reactor vessel 12. A series of spaced apart top member openings 16.2 are provided proximate the outer edges of the rectangular top member 16.

The top member 16 is made from a polyester and vinyl composite 16 which has been treated for acid and UV resistance. The anaerobic digester assembly 10 further comprises sealing means 22 for sealing the top member 16 onto the bottom member 14, in particular seal the top member 16 in the channel 33 of the seat 30 of the bottom member 14.

The sealing means 22 comprises a first seal member 22.2 which is in the form of a rectangular frame so as to conform to the shape of the seat 30 and channel 33. The sealing means 22 also comprises a second seal member 22.4 which is the same shape as the first seal member 22.2. Each of the first and second seal members 22.2 and 22.4 respectively, define a series of spaced apart seal member openings 22.6. It will be appreciated that the shape of the first and second seal means 22.2 and 22.4 respectively, may differ according to the shape and configuration of the reactor vessel 12. The first seal member 22.2 and the second seal member 22.4 are generally rubber gaskets.

As shown in the Figures 1 and 3 to 5 of the drawings, the anaerobic digester assembly 10 further comprises fastening/securing means 24. The securing means 24 (Figure 1A) is provided for securing the top member 16 onto the base member 14 as will be described in more detail below. The securing means comprises a threaded bolt 24.2, an upper nut 24.4 and a lower nut 24.6.

As shown in Figure 2, the anaerobic digester assembly 10 further comprises a baffle 26 that extends inwardly across the base 14.10 of the base member 14 and is arranged transverse to the longitudinal axis A-A of the base member 14 . The baffle 26 is disposed proximate the first end wall 14.6 of the base member 14. The baffle 24 is arranged to act as a weir to enhance mixing of the organic waste received in the inlet end 18 in the space defined between the baffle 26 and the first end wall 14.6 and further to avoid new organic waste from migrating into the space on the other side of the baffle 26 on the outlet 20 side before complete digestion of the organic waste.

The anaerobic digester assembly 10 further comprises a drainage hole 28 defined by the base 14.10 of the base member 14. The drainage hole 28 is disposed proximate the digestate outlet 20. The drainage hole 28 may be used for draining digestate or organic waste material during periodical servicing and cleaning of the reactor vessel 12.

The anaerobic digester assembly 10 further comprises an organic waste receptacle 32 that is flush fitted to the first end wall 14.6 and has an opening that is in register and communication with the inlet 18 on the first end wall 14.6. The organic waste receptacle 32 is fitted with a lid 34. The organic waste receiving receptacle 32 is typically arranged to feed organic waste into the base member 14 under gravity and the organic waste is arranged to move steadily within the base member 14 and over the baffle 26 under gravity owing to the angle of inclination of the base 14.10 relative to the horizontal from the first end wall 14.6 side to the second end wall 14.8 side.

The anaerobic digester assembly 10 further comprises a digestate holding receptacle 36 that is flush fitted to the second end wall 14.8 and has an opening that is in register and communication with the outlet 20 on the second end wall 14.8. The digestate holding receptacle 36 is fitted with a lid 38. The anaerobic digester assembly 10 further comprises an arrangement of attachment means in the form of brackets 40 each fitted to a respective corner of the base member 14. Each bracket 40 is arranged to be attached and secured to a removable greenhouse cover (not shown).The greenhouse cover (not shown) is arranged to trap heat generated from the reactor vessel 12 of the anaerobic digester assembly 10, which heat can be circulated back into the reactor vessel 12, typically via the top member 16, to facilitate further digestion of the organic waste.

The anaerobic digester assembly 10 further comprises an outer frame 15 which is also in the shape of the first and second seal members 22.2 and 22.4 respectively. The outer frame 15 has an outwardly projecting frame lip 15.1 that is arranged to abut against the lip of the seat 30 once secured thereto. The outer frame 15 defines a series of spaced apart outer frame openings 15.2 on the frame lip 15.1.

In use, as shown more clearly in Figure 5, the first seal member 22.2 is accommodated in the channel 33 of the seat 30 and the first seal member openings 22.6 are arranged in register with the flange openings 30.2 of the seat 30. The top member 16 is then placed on top of the first seal member 22.2. In pacing the top member on the first seal member 22.2, the top member openings 16.2 are arranged in register with the first seal member openings 22.6 and a major portion of the top member 16 covers the upper opening 14.12 of the base member 14, as shown in Figure 1. The second seal member 22.4 is then placed on top of the top member 16 such that the second seal member openings 22.6 are also in register with the top member openings 16.2. The outer frame 15 is then placed on top of the second seal member 22.4 and the outer frame openings 15.2 are arranged in register with second seal openings 22.6 which are in register with the flange openings 30.2. A series of dedicated bolts 24.2 are then passed through the channel defined by the aligned openings 15.2, 22.6, 16.2, 22.6, 30.2 of the sequential arrangement of the outer frame 15, second seal member 22.4, top member 16, first seal member 22.2 and the base member 14, respectively. An upper nut 24.4 is threaded on an upper threaded portion of the bolt 24.2 and a lower nut 24.6 is threaded on a lower threaded portion of the bolt 24.2 to secure the outer frame 15 to the base member 14 which results in the top member being securely sealed between the first and second seal members 22.2 and 22.4 respectively. During digestion, the top member 16, which is secured to the base member 14 expands as shown in Figure 4 due to the pressure of expanding gases formed during digestion of the organic waste.

The anaerobic digester assembly 10 is simple in design, durable, produced from low cost materials, compact and portable. The drainage hole 28 provided in the base member 14 also allows the reactor vessel 12 to be maintained and cleaned when fouling is experienced.

## Claims

1. An anaerobic digester assembly (10) comprising:
a horizontally arranged elongate reactor vessel (12) having an elongate base member (14), and a resiliently flexible top member (16) covering an operatively upper open end (14.12) of the base member, wherein
the resiliently flexible top member being sealed and secured relative to the base member,
wherein the base member defining an organic waste inlet at a first end thereof and a digestate outlet at a second end thereof, **characterized in that** the elongate base member is made from a thermoplastic polymer; a base of the base member is inclined between the inlet and outlet to allow organic feed and digestate to flow under the influence of gravity, in use; and
at least one baffle member (26) is arranged to act as a barrier to promote mixing of the feed before flowing over the at least one baffle, wherein the at least one baffle member is disposed on the base and arranged to extend at least partly across the base, wherein the at least one baffle member is arranged transversely relative to a longitudinal axis of the base, and further wherein the at least one baffle member is disposed near the inlet.

2. The anaerobic digester assembly according to claim-1, wherein the base member defining a drainage hole that is disposed proximate the outlet.

3. The anaerobic digester assembly according to any one of preceding claims, wherein the base of the base member is arranged at an inclination of at least about 2 degrees relative to the horizontal.

4. The anaerobic digester assembly according to any one of the preceding claims, wherein the base member comprises a seat that projects laterally outwardly from the base member.

5. The anaerobic digester assembly according to claim 4, wherein the seat defines a channel and has an outer lip defining a plurality of laterally spaced apart openings.

6. The anaerobic digester assembly according to any one of the preceding claims, including a sealing means for providing an airtight configuration between the top member and the base member.

7. The anaerobic digester assembly according to claim 6 when dependent on claim 5, wherein the sealing means including an operatively upper first seal member, and an operatively lower second seal member accommodated by the channel, wherein an outer, peripheral portion of the top member is sandwiched between the upper first seal member and the lower second seal member.

8. The anaerobic digester assembly according to claim 7, including an outer frame abutting the upper first seal member.

9. The anaerobic digester assembly according to claim 8, wherein the outer frame, the upper first seal member and the lower second seal member each define spaced apart openings which are in register with the openings on the outer lip of the seat.

10. The anaerobic digester assembly according to any one of claims 5 to 9, comprising securing means for securing the top member to the base member.

11. The anaerobic digester assembly according claim 10 when dependent on claim 9, wherein the securing means including a plurality of bolts, each bolt being inserted in a channel defined by each of the aligned openings of the outer frame, upper first seal member, top member, lower second seal member and the openings on the outer lip of the seat of the base member, and wherein the securing means further including a plurality of operatively upper nuts and operatively lower nuts for securing the plurality of bolts to the outer frame, upper first seal member, top member, lower second seal member and the base member.

12. The anaerobic digester assembly according to any one of the preceding claims, wherein the thermoplastic polymer is selected from a polypropylene, polyvinyl chloride, High Density Polyethylene (HDPE).

13. The anaerobic digester assembly according to any one of the preceding claims, wherein the resiliently flexible top member is made from a composite material comprising a polyester and a vinyl.

14. The anaerobic digester assembly according to claim 13, wherein the composite material is treated for acid and UV resistance.

## Patentansprüche

1. Anaerobe Faulbehälteranordnung (10), umfassend:
einen horizontal angeordneten länglichen Reaktorbehälter (12) mit einem länglichen Basiselement (14) und einem elastisch flexiblen Oberteil (16), das ein operativ oberes offenes Ende (14.12) des Basiselements bedeckt, wobei
das elastisch flexible Oberteil abgedichtet ist und in Bezug auf das Basiselement gesichert ist;
wobei das Basiselement einen Einlass für organische Abfälle an einem ersten Ende davon und einen Auslass für Faulrückstände an einem zweiten Ende davon definiert, **dadurch gekennzeichnet, dass** das längliche Basiselement aus einem thermoplastischen Polymer angefertigt ist; eine Basis des Basiselements zwischen Einlass und Auslass geneigt ist, um organisches Einsatzmaterial und Faulrückstände unter dem Einfluss der Schwerkraft fließen zu lassen; und
mindestens ein Leitelement (26) so angeordnet ist, dass es als eine Barriere fungiert, um Mischen des Einsatzmaterials vor Fließen über das mindestens eine Leitelement zu fördern, wobei das mindestens eine Leitelement auf der Basis angeordnet ist und so angeordnet ist, dass es sich mindestens teilweise über die Basis hinweg erstreckt, wobei das wenigstens eine Leitelement quer zu einer Längsachse der Basis angeordnet ist, und wobei ferner das wenigstens eine Leitelement nahe am Einlass angeordnet ist.

2. Anaerobe Faulbehälteranordnung nach Anspruch 1, wobei das Basiselement ein Abflussloch definiert, das in der Nähe des Auslasses angeordnet ist.

3. Anaerobe Faulbehälteranordnung nach einem der vorangehenden Ansprüche, wobei die Basis des Basiselements in einer Neigung von mindestens etwa 2 Grad relativ zur Horizontalen angeordnet ist.

4. Anaerobe Faulbehälteranordnung nach einem der vorhergehenden Ansprüche, wobei das Basiselement einen Sitz umfasst, der seitlich nach außen aus dem Basiselement hinausragt.

5. Anaerobe Faulbehälteranordnung nach Anspruch 4, wobei der Sitz einen Kanal definiert und eine äußere Lippe aufweist, die eine Vielzahl von seitlich voneinander beabstandeten Öffnungen definiert.

6. Anaerobe Faulbehälteranordnung nach einem der vorhergehenden Ansprüche, ein Abdichtmittel zum Bereitstellen einer luftdichten Konfiguration zwischen dem Oberteil und dem Basiselement umfassend.

7. Anaerobe Faulbehälteranordnung nach Anspruch 6, wenn abhängig von Anspruch 5, wobei das Abdichtmittel ein operativ oberes erstes Abdichtelement und ein operativ unteres zweites Abdichtelement umfasst, das von dem Kanal aufgenommen wird, wobei ein äußerer Umfangsabschnitt des Oberteils zwischen dem oberen ersten Abdichtelement und dem unteren zweiten Abdichtelement eingefasst ist.

8. Anaerobe Faulbehälteranordnung nach Anspruch 7, einen äußeren Rahmen umfassend, der an das obere erste Abdichtelement anschließt.

9. Anaerobe Faulbehälteranordnung nach Anspruch 8, wobei der äußere Rahmen, das obere erste Abdichtelement und das untere zweite Abdichtelement jeweils beabstandete Öffnungen definieren, die mit den Öffnungen auf der äußeren Lippe des Sitzes in Deckung sind.

10. Anaerobe Faulbehälteranordnung nach einem der Ansprüche 5 bis 9, ein Sicherungsmittel zur Sicherung des Oberteils am Basiselement umfassend.

11. Anaerobe Faulbehälteranordnung nach Anspruch 10, wenn abhängig von Anspruch 9, wobei das Sicherungsmittel eine Vielzahl von Schrauben umfasst, wobei jede Schraube in einen Kanal eingeführt ist, der durch jede der ausgerichteten Öffnungen des äußeren Rahmens, des oberen ersten Abdichtelements, des Oberteils, des unteren zweiten Abdichtelements und der Öffnungen auf der äußeren Lippe des Sitzes des Basiselements definiert ist, und wobei das Sicherungsmittel weiterhin eine Vielzahl von operativ oberen Muttern und operativ unteren Muttern zur Sicherung der Vielzahl von Schrauben am äußeren Rahmen, am oberen ersten Abdichtelement, am Oberteil, am unteren zweiten Abdichtelement und am Basiselement umfasst.

12. Anaerobe Faulbehälteranordnung nach einem der vorhergehenden Ansprüche, wobei das thermoplastische Polymer aus einem Polypropylen, Polyvinylchlorid, Polyethylen hoher Dichte (HDPE) ausgewählt ist.

13. Anaerobe Faulbehälteranordnung nach einem der vorhergehenden Ansprüche, wobei das elastisch flexible Oberteil aus einem Verbundmaterial angefertigt ist, das einen Polyester und ein Vinyl umfasst.

14. Anaerobe Faulbehälteranordnung nach Anspruch 13, wobei das Verbundmaterial für Säure- und UV-Beständigkeit behandelt ist.

## Revendications

1. Ensemble digesteur anaérobie (10) comprenant :
une cuve de réaction allongée disposée horizontalement (12) comportant un élément de base allongé (14), et un élément supérieur flexible élastiquement (16) recouvrant une extrémité supérieure ouverte fonctionnelle (14.12) de l'élément de base,
l'élément supérieur flexible élastiquement étant scellé et fixé à l'élément de base,
l'élément de base définissant une entrée de déchets organiques à une première extrémité de celui-ci et une sortie de digestat à une deuxième extrémité de celui-ci, **caractérisé en ce que** l'élément de base allongé est fabriqué à partir d'un polymère thermoplastique ; une base de l'élément de base est inclinée entre l'entrée et la sortie pour permettre à la charge organique et au digestat de s'écouler sous l'influence de la gravité à l'usage ; et
au moins un élément déflecteur (26) est agencé pour agir comme barrière afin de favoriser le mélange de la charge avant qu'elle s'écoule par-dessus l'au moins un déflecteur, l'au moins un élément déflecteur étant disposé sur la base et agencé pour s'étendre au moins partiellement à travers la base, l'au moins un élément déflecteur étant disposé transversalement par rapport à un axe longitudinal de la base, et l'au moins un élément déflecteur étant en outre disposé près de l'entrée.

2. Ensemble digesteur anaérobie selon la revendication 1, dans lequel l'élément de base définit un trou de drainage qui est disposé à proximité de la sortie.

3. Ensemble digesteur anaérobie selon l'une quelconque des revendications précédentes, dans lequel la base de l'élément de base est disposée avec une inclinaison d'au moins 2 degrés environ par rapport à l'horizontale.

4. Ensemble digesteur anaérobie selon l'une quelconque des revendications précédentes, dans lequel l'élément de base comprend une assise qui fait saillie latéralement vers l'extérieur depuis l'élément de base.

5. Ensemble digesteur anaérobie selon la revendication 4, dans lequel l'assise définit un canal et comporte une lèvre extérieure définissant une pluralité d'ouvertures espacées latéralement.

6. Ensemble digesteur anaérobie selon l'une quelconque des revendications précédentes, comportant des moyens d'étanchéité destinés à assurer une configuration étanche à l'air entre l'élément supérieur et l'élément de base.

7. Ensemble digesteur anaérobie selon la revendication 6 lorsqu'elle est dépendante de la revendication 5, dans lequel les moyens d'étanchéité comportent un premier élément d'étanchéité supérieur fonctionnel et un deuxième élément d'étanchéité inférieur fonctionnel accueilli par le canal, une partie périphérique extérieure de l'élément supérieur étant intercalée entre le premier élément d'étanchéité supérieur et le deuxième élément d'étanchéité inférieur.

8. Ensemble digesteur anaérobie selon la revendication 7, comportant une armature extérieure jouxtant le premier élément d'étanchéité supérieur.

9. Ensemble digesteur anaérobie selon la revendication 8, dans lequel l'armature extérieure, le premier élément d'étanchéité supérieur et le deuxième élément d'étanchéité inférieur définissent chacun des ouvertures espacées qui sont alignées avec les ouvertures sur la lèvre extérieure de l'assise.

10. Ensemble digesteur anaérobie selon l'une quelconque des revendications 5 à 9, comprenant des moyens de fixation destinés à fixer l'élément supérieur à l'élément de base.

11. Ensemble digesteur anaérobie selon la revendication 10 lorsqu'elle est dépendante de la revendication 9, dans lequel les moyens de fixation comportent une pluralité de boulons, chaque boulon étant inséré dans un canal défini à la fois par les ouvertures alignées du cadre extérieur, le premier élément d'étanchéité supérieur, l'élément supérieur, le deuxième élément d'étanchéité inférieur et les ouvertures sur la lèvre extérieure de l'assise de l'élément de base, et dans lequel les moyens de fixation comportent en outre une pluralité d'écrous supérieurs fonctionnels et d'écrous inférieurs fonctionnels destinés à fixer la pluralité de boulons à l'armature extérieure, au premier élément d'étanchéité supérieur, à l'élément supérieur, au deuxième élément d'étanchéité inférieur et à l'élément de base.

12. Ensemble digesteur anaérobie selon l'une quelconque des revendications précédentes, dans lequel le polymère thermoplastique est choisi parmi le polypropylène, le polychlorure de vinyle et le polyéthylène haute densité (HDPE).

13. Ensemble digesteur anaérobie selon l'une quelconque des revendications précédentes, dans lequel l'élément supérieur flexible élastiquement est fabriqué à partir d'un matériau composite comprenant un polyester et un vinyle.

14. Ensemble digesteur anaérobie selon la revendication 13, dans lequel le matériau composite est traité pour la résistance aux acides et aux UV.
